Europäisches Patentamt

European Patent Office

Office européen des brevets

(19)

(11) Publication number: **0 236 949**
**A2**

## (12) EUROPEAN PATENT APPLICATION

(21) Application number: **87103128.2**

(22) Date of filing: **05.03.87**

(51) Int. Cl.³: **A 61 K 35/18**
**A 61 K 47/00**

(30) Priority: **07.03.86 JP 51055/86**

(43) Date of publication of application:
**16.09.87 Bulletin 87/38**

(84) Designated Contracting States:
**AT BE CH DE ES FR GB GR IT LI LU NL SE**

(71) Applicant: **Takeda Chemical Industries, Ltd.**
**27, Doshomachi 2-chome Higashi-ku**
**Osaka-shi Osaka, 541(JP)**

(72) Inventor: **Imada, Isuke**
**18-10, Tsuruyamadai 4-chome**
**Izumi Osaka 594(JP)**

(74) Representative: **von Kreisler, Alek, Dipl.-Chem. et al,**
**Deichmannhaus am Hauptbahnhof**
**D-5000 Köln 1(DE)**

(54) **Blood preservation.**

(57) The physiological activity of red blood cells is prolonged when stored by an aqueous solution containing oxalic acid or its salt. Therefore, a composition for blood preservation containing oxalic acid or its salt are useful for long-term storage of blood, especially red blood cells.

EP 0 236 949 A2

BLOOD PRESERVATION

## Summary of the Invention

This invention relates to a method for blood preservation. More particularly, this invention relates to a method for preserving blood or red blood cells characterized by adding the blood or red blood cells in an aqueous solution for blood preservation containing oxalic acid or its salt, and to a composition to be used for the preservation of blood or red blood cells.

## Background Art

In animals including human, blood is essential to the energy metabolism in tissues and cells. Especially, the red blood cell as a blood component is essential to the supply of oxygen to tissues and cells. Animals with excessive bleeding due to surgical operation, traffic accident and the like are therefore in danger of death, and blood transfusion is usually carried out to save their life. In addition, componential blood transfusion using packed red blood cells and the like are carried out in the case of hypoplastic anemia, bleeding due to hepatargia, surgical operations, chronic renal diseases and replacement transfusion.

Blood to be transfused is collected from healthy animals and stored before use, and various solutions for blood preservation or red blood cell preservation are used because the term of validity of stored blood is limited. These solutions for preservation are isotonic without exception, but ACD solution, CPD solution, ADSOL® solution and the like are generally used.

Even if stored at 4 to 6°C using such a solution for blood preservation, neither blood nor red blood cell remains valid for more than 3 weeks. Specifically in the case of humans, it is often necessary to choose suitable donors by reasons of problems such as blood group incompatibility. It is therefore desired that a solution for blood preservation that enables the long-term storage of blood, especially red blood cells, will be developed.

Disclosure of the Invention

The present invention provides (1) a method for preserving blood or red blood cells, characterized by suspending the blood or red blood cells in an aqueous solution for blood preservation containing oxalic acid or a physiologically acceptable water-soluble salt thereof to render a concentration of oxalic acid or a physiologically acceptable water-soluble salt thereof in the prepared suspension 0.1 to 1 mM, (2) an improved composition for preservation of blood or red blood cells containing an anticoagulant or a preservative for red blood cells, the improvement which comprises incorporating oxalic acid or a physiologically acceptable water-soluble salt thereof to the composition to maintain the physiological activity of blood or red blood cells, and (3) a suspension of red blood cells in the aqueous solution for blood preservation.

As examples of the physiologically acceptable salt of oxalic acid, there may be mentioned alkali metal salts of oxalic acid such as sodium oxalate and potassium oxalate, but oxalic acid is preferably used.

Substances other than either oxalic acid or its salt which are used as components of the composition for blood preservation include per se known anticoagulants for blood preservation such as ACD (2.2 weight parts of sodium citrate, 0.8 weight part of citric acid and 2.45 weight parts of glucose), CPD (2.57 weight parts of sodium citrate, 0.31 weight part of citric acid, 0.21 weight part of sodium dihydrogen phosphate and 2.5 weight parts of glucose), ACD-adenine (0.047 weight part of adenine is added to 5.45 weight parts of above ACD) and CPD-adenine (adenine is added to above CPD), and per se known preservatives for red blood cells such as ADSOL® [2.0 weight parts of glucose, 0.95 weight part of sodium chloride (or 2.2 weight parts of glucose, 0.90 weight part of sodium chloride), 0.75 weight part of mannitol and 0.027 weight part of adenine].

Oxalic acid and/or its physiologically acceptable salt is dispensed to the composition for blood preservation so that the concentration of oxalic acid and/or its salt can be 0.1 mM to 1 mM, preferably 0.1 mM to 0.4 mM, in a blood preservation mixture prepared by suspending blood or red blood cells in an aqueous solution for blood preservation. As examples of such formulations, there may be mentioned (i) 2.2 weight parts of sodium citrate, 0.8 weight part of citric acid, 2.45 weight parts of glucose and 0.007 to 0.07 weight part of oxalic acid and/or its salt (hereinafter referred to as ACD-oxalic acid), (ii) 2.57 weight parts of sodium citrate, 0.31 weight part of citric acid, 0.21 weight part of sodium dihydrogen phosphate, 2.5 weight parts of glucose and 0.007 to 0.07 weight part of oxalic acid and/or its salt (hereinafter referred to as CPD-oxalic acid), (iii) 2.2 weight parts of sodium citrate, 0.8 weight part of citric acid, 2.45 weight parts of glucose, 0.047 weight parts of adenine and 0.007 to 0.07 weight part of oxalic acid and/or its salt (hereinafter referred to as ACD-adenine-oxalic acid) and (iv) 2.0 weight parts of glucose, 0.95 weight part of sodium chloride (or 2.2 weight parts of glucose, 0.90 weight part of sodium chloride), 0.75 weight part of mannitol, 0.027 weight part of adenine and 0.0018 to 0.018 weight part of oxalic acid and/or its salt (hereinafter referred to as ADSOL-oxalic acid). The above amounts of oxalic acid and/or its salt to be dispensed can vary with the sort of the object to be
preserved (blood or red blood cells) and the volume of the
aqueous solution for blood preservation to be used for the
object to be preserved. Other substances which do not
interfere with the preservation of blood or red blood
cells, such as ascorbic acid and its derivatives (e.g.
magnesium ascorbate phosphate) may also be added.

The composition for blood preservation of the present
invention may be solid (powder, granules, etc.) or liquid
(aqueous solution, etc.). When prepared as a liquid
composition, it is not necessarily an isotonic solution,
and may be a hypertonic solution. When prepared as a solid
composition, it can be diluted with distilled water for
injection to prepare an isotonic solution before use. When
prepared as a hypertonic solution, it can be diluted with
distilled water for injection to prepare an isotonic
solution before use.

The amount of distilled water for injection used for
preparation of an aqueous solution for blood preservation
varies with the sort and amount of the anticoagulant or the
preservative for red blood cells to be used and the amount
of oxalic acid and/or its salt to be used, but, for
example, it is preferable that 5.46-5.52 g of ACD-oxalic
acid, 5.60-5.66 g of CPD-oxalic acid, 5.50-5.57 g of ACD-
adenine-oxalic acid or 3.73-3.75 g of ADSOL-oxalic acid be
diluted with distilled water for injection to a total
volume of 100 mℓ.

The preparation of the composition for blood
preservation and red blood cell suspension of the present
invention, including the distribution of oxalic acid or its
salt to the anticoagulant for blood preservation or the
preservative for red blood cells can be achieved using
conventional methods.

The amount of aqueous solution for blood preservation used for storage of blood varies with the sort and amount of the aqueous solution to be used; for example, it is preferable that 75 m$\ell$ of the ACD-oxalic acid solution or ACD-adenine-oxalic acid solution or 70 m$\ell$ of the CPD-oxalic acid solution can be used for each 500 m$\ell$ of blood. The amount of aqueous solution for blood preservation used for storage of red blood cells varies with the sort and amount of the aqueous solution; for example, when the ADSOL-oxalic acid solution is used, it is preferable that 100 m$\ell$ of the ADSOL-oxalic acid solution is used to 100 m$\ell$ of ADSOL solution in which red blood cells are suspended, so that red blood cells can be suspended in the prepared ADSOL-oxalic acid suspension to a concentration of $5 \times 10^{11}$ to $5 \times 10^{13}$ cells (more preferably $1 \times 10^{12}$ to $1 \times 10^{13}$ cells, further preferably $4 \times 10^{12}$ to $6 \times 10^{12}$ cells) per 1 $\ell$ of the suspension. When red blood cells are preserved using an anticoagulant for blood preservation such as ACD, ACD-adenine, CPD or CPD-adenine, oxalic acid or its salt is similarly dissolved in an aqueous solution for blood preservation to a final concentration of 0.1 to 1 mM.

The composition for blood preservation of the present invention can be similarly used for preservation of blood or red blood cells of warm-blooded animals besides human (e.g. sheep, dog, hen, rat, mouse). It is preferable that oxalic acid and/or a physiologically acceptable salt thereof is dispensed to the composition for blood preservation so that the concentration of oxalic acid and/or its salt can be 0.1 mM to 1 mM, preferably 0.1 mM to 0.4 mM, in a blood preservation mixture prepared by suspending blood or red blood cells in an aqueous solution for blood preservation. Referring to substances other than oxalic acid or its salt as components of the composition for blood preservation, there are used anticoagulants and preservatives for red blood cells which are suitable for preservation of blood or red blood cells of each animal. For the purpose of blood preservation of rat, for example, it is preferable that 100 mℓ of Alsever-oxalic acid solution [prepared by adding 0.2 to 2.0 mM of oxalic acid or its salt to Alsever's solution comprising 20.5 g of glucose, 4.2 g of sodium chloride, 8.0 g of sodium citrate and suitable amount (about 0.63 g) of citric acid per 1 ℓ (pH 6.1)] is used to 100 mℓ of blood of rat.

It is usually preferable that the storage of blood or red blood cells using the aqueous solution for blood preservation be at 4 to 6 °C.

The physiological activity of red blood cells is prolonged when stored by an aqueous solution containing oxalic acid or its salt.

The composition for blood preservation of the present invention is useful for long-term storage of blood, specifically red blood cells.

The present invention is hereinafter illustrated more concretely with some examples, but the present invention should not be limited thereto.

Example 1

Two (2) g of glucose, 0.95 g of sodium chloride, 0.75 g of mannitol, 0.027 g of adenine and 1.8 mg of oxalic acid are dissolved in distilled water to a total volume of 100 mℓ, then the solution is sterilized by filtration using a sterile filter to prepare a 0.2 mM oxalic acid-ADSOL® solution. The resulting solution is added to an equal volume of a suspension of red blood cells in ADSOL®.

Example 2

Using the same procedure as in Example 1 except for using 7.2 mg of oxalic acid, a 0.8 mM oxalic acid-ADSOL® solution is prepared. The resulting solution is added to an equal volume of a suspension of red blood cells in ADSOL®.

Example 3

In distilled water are dissolved 20.5 g of glucose, 4.2 g of sodium chloride and 8.0 g of sodium citrate, and a suitable amount of citric acid and distilled water are added to the mixture to prepare 1 ℓ of Alsever's solution having a pH value of 6.1. To this solution is added 72 mg of oxalic acid and the resulting mixture is sterilized by filtration to prepare 0.8 mM oxalic acid-Alsever's solution. The prepared solution is added to an equal volume of blood of rat (or citric acid-addition rat blood).

Example 4

In distilled water are dissolved 26.3 g of sodium citrate, 3.27 g of citric acid, 23.2 g of glucose, 2.51 g of sodium dihydrogen phosphate (dihydrate), 55 mg of adenine and 29 mg of oxalic acid to a total volume of 100 mℓ. This solution is sterilized by filtration to prepare concentrated CPD-adenine-oxalic acid solution. Fourteen (14) mℓ of this solution may be added to 100 mℓ of blood.

The present invention is hereinafter illustrated in more detail with experimental examples.

Experimental Example 1

About 0.5 mℓ of red blood cells collected from a healthy adult human was washed with about a 10-fold volume of ADSOL® (isotonic solution composed of sodium chloride, glucose, adenine and mannitol) to obtain 1 mℓ of an ADSOL suspension [hemoglobin content: about 0.1 g/mℓ[1)]]. To this

suspension, 1 mℓ of 0.2 mM or 0.8 mM oxalic acid-ADSOL® solution was added to prepare a sample. The retention rate of red blood cell function was determined by measuring the amounts of 2,3-diphosphoglycerate[2] in the red blood cells. For comparison, EDTA-addition solutions were also examined, which were thought to inhibit blood coagulation via binding with $Ca^{2+}$ ions in blood plasma. As a result, as shown in Table 1, it was found that when stored in a solution containing oxalic acid, red blood cells hold their function for a longer period than in an ordinary solution for blood storage.

1) The cyanmethemoglobin contents were determined using the hemoglobin assay kit "Hemoglobin-Test-Wako", manufactured by Wako Pure Chemical.

2) Nishina, H.: Rinsho Kensa Gijutsu Zensho Vol. 3, Ketsueki Kensa (Book for Medical Technology, vol. 3, Hemanalysis), edited by Shiro Miura, Tokyo, Igaku Shoin, 1972, pp. 324-325.

Table 1   2,3-Diphosphoglycerate Contents in Red Blood Cells

| Compound Added to ADSOL Solution (Final Concentration, mM) | 2,3-Diphosphoglycerate Content (µmol/g Hemoglobin) | | | | |
|---|---|---|---|---|---|
| | 2nd Day | 8th Day | 15th Day | 21st Day | 36th Day |
| Not added | 10.5 | 12.5 | 4.1 | 1.0 | 0.5 |
| EDTA2Na (0.4) | 11.0 | 8.0 | 2.2 | 0 | 0.5 |
| EDTA2Na (0.1) | 10.5 | 9.0 | 3.6 | 0.5 | 0.3 |
| Oxalic acid (0.4) | 9.0 | 13.5 | 16.4 | 14.0 | 10.7 |
| Oxalic acid (0.1) | 10.5 | 14.5 | 14.6 | 8.5 | 4.5 |

As for substances similar to oxalic acid, 2,3-diphosphoglycerate contents were also determined in the same manner as above to examine oxalic acid for specific positive effect on the retention of red blood cell function.  As a result, as shown in Table 2, it was proved that the effect was specific to oxalic acid.

Table 2 Effect of Various Acids on the 2,3-Diphospho-glycerate Contents in Red Blood Cells

| Compound Added to ADSOL Solution (Final Concentration, mM | 2,3-Diphosphoglycerate Content (μmol/g Hemoglobin) | | |
|---|---|---|---|
| | 0th Day | 7th Day | 14th Day |
| Not added | 14.2 | 18.8 | 10.5 |
| Oxalic acid (0.1) | 15.0 | 20.5 | 19.8 |
| Citric acid (0.1) | 15.8 | 18.8 | 10.8 |
| Succinic acid (0.1) | 15.2 | 18.9 | 10.2 |
| Malonic acid (0.1) | 14.6 | 19.6 | 10.9 |

Experimental Example 2

From a rat (male Wister, 10-12 weeks of age), 9 mℓ of blood was collected and immediately added to 1 mℓ of citric acid to prepare blood suspension. To this suspension is added 10 mℓ of 0.8 mM oxalic acid-Alsever's solution, and the resulting mixture was stored at 4°C while 2,3-diphosphoglycerate contents in red blood cells were measured. The same procedure was applied to Alsever's solution not containing oxalic acid.

As a result, as shown in Table 3, it was found that when stored in Alsever's solution containing oxalic acid, red blood cells hold their function for longer period than in Alsever's solution to which oxalic acid was not added.

Table  3 2,3-Diphosphoglycerate Contents in Rat

Red Blood Cells

| Compound Added to Alsever's Solution (Final Concentration, mM) | 2,3-Diphosphoglycerate Content (μmol/g Hemoglobin) | | |
|---|---|---|---|
| | 0th Day | 8th Day | 19th Day |
| Not added | 24.5 | 3.9 | 0.7 |
| Oxalic acid (0.4) | 22.5 | 22.5 | 14.3 |

Experimental Example 3

From a healthy adult human, 7 mℓ of blood was collected and immediately 0.98 mℓ of CPD-adenine-oxalic acid solution prepared in Example 4 was added to the blood. The resulting mixture was stored at 4°C while 2,3-diphosphoglycerate contents in red blood cells were measured.  The same procedure was applied to concentrated CPD-adenine solution not containing oxalic acid.

As a result, as shown in Table 4, it was found that when stored in CPD-adenine solution containing oxalic acid, red blood cells hold their function for longer period.

Table 4      2,3-Diphosphoglycerate Contents in Red Blood Cells

| Compound Added to CPD-adenine Solution (Final Concentration, mM) | 2,3-Diphosphoglycerate ($\mu$mol/g Hemoglobin) | | |
|---|---|---|---|
| | 0th Day | 8th Day | 15th Day |
| Not added | 11.6 | 1.1 | 0.6 |
| Oxalic acid (0.4) | 13.3 | 7.4 | 5.1 |

What is claimed is:

1. A method for preserving red blood cells, characterized by suspending said red blood cells in an aqueous solution for blood preservation containing oxalic acid or a physiologically acceptable water-soluble salt thereof to render a concentration of oxalic acid or a physiologically acceptable water-soluble salt thereof in the prepared red blood cells suspension 0.1 to 1 mM.

2. A method according to claim 1, wherein the concentration of oxalic acid or a physiologically acceptable water-soluble salt thereof in the prepared suspension is 0.1 to 0.4 mM.

3. A method according to claim 1, wherein the aqueous solution for blood preservation comprises (1) glucose, (2) sodium chloride, (3) mannitol, (4) adenine and (5) oxalic acid or a physiologically acceptable water-soluble salt thereof.

4. A method according to claim 1, wherein the aqueous solution for blood preservation comprises (1) sodium citrate, (2) citric acid, (3) glucose and (4) oxalic acid or a physiologically acceptable water-soluble salt thereof.

5. A method according to claim 1, wherein the aqueous solution for blood preservation comprises (1) sodium citrate, (2) citric acid, (3) sodium dihydrogen phosphate, (4) glucose and (5) oxalic acid or a physiologically acceptable water-soluble salt thereof.

6. A method according to claim 1, wherein the aqueous solution for blood preservation comprises (1) sodium citrate, (2) citric acid, (3) glucose, (4) adenine and (5) oxalic acid or a physiologically acceptable water-soluble salt thereof.

7. A method according to claim 1, wherein the aqueous solution for blood preservation comprises (1) sodium citrate, (2) citric acid, (3) sodium dihydrogen phosphate, (4) glucose, (5) adenine and (6) oxalic acid or a physiologically acceptable water-soluble salt thereof.

8. A method according to claim 1, wherein the aqueous solution for blood preservation comprises (1) glucose, (2) sodium chloride, (3) sodium citrate, (4) citric acid and (5) oxalic acid or a physiologically acceptable water-soluble salt thereof.

9. A method for preserving blood, characterized by adding an aqueous solution for blood preservation containing oxalic acid or a physiologically acceptable water-soluble salt thereof to said blood to render a concentration of oxalic acid or a physiologically acceptable water-soluble salt thereof in the prepared blood suspension 0.1 to 1 mM.

10. A method according to claim 9, wherein the concentration of oxalic acid or a physiologically acceptable water-soluble salt thereof in the prepared blood suspension is 0.1 to 0.4 mM.

11. An improved composition for preservation of blood or red blood cells containing an anticoagulant or a preservative for red blood cells, the improvement which comprises incorporating oxalic acid or a physiologically acceptable water-soluble salt thereof to the composition to maintain the physiological activity of blood or red blood cells.

12. A suspension of red blood cells in an aqueous solution for blood preservation containing oxalic acid or a physiologically acceptable water-soluble salt thereof of which the concentration is 0.1 to 1 mM.